Europäisches Patentamt

European Patent Office

Office européen des brevets

(11) Publication number : **0 408 918 B1**

# (12) EUROPEAN PATENT SPECIFICATION

(45) Date of publication of patent specification :
**18.11.93 Bulletin 93/46**

(51) Int. Cl.⁵ : **C12Q 1/68, // C07H21/04**

(21) Application number : **90111773.9**

(22) Date of filing : **21.06.90**

(54) **Method for detecting nucleic acid.**

(30) Priority : **23.06.89 JP 159718/89**
**24.11.89 JP 303182/89**

(43) Date of publication of application :
**23.01.91 Bulletin 91/04**

(45) Publication of the grant of the patent :
**18.11.93 Bulletin 93/46**

(84) Designated Contracting States :
**AT BE CH DE DK ES FR GB GR IT LI LU NL SE**

(56) References cited :
**EP-A- 0 135 159**
**EP-A- 0 144 914**
**EP-A- 0 146 815**
**EP-A- 0 235 726**
**WO-A-86/03227**
**US-A- 4 851 331**

(73) Proprietor : **CANON KABUSHIKI KAISHA**
**30-2, 3-chome, Shimomaruko, Ohta-ku**
**Tokyo (JP)**

(72) Inventor : **Kato, Kinya, c/o Canon K.K.**
**30-2, 3-chome**
**Shimomaruko, Ohta-ku, Tokyo (JP)**
Inventor : **Iwashita, Harumi, c/o Canon K.K.**
**30-2, 3-chome**
**Shimomaruko, Ohta-ku, Tokyo (JP)**
Inventor : **Yamamoto, Nobuko, c/o Canon K.K.**
**30-2, 3-chome**
**Shimomaruko, Ohta-ku, Tokyo (JP)**
Inventor : **Sakuranaga, Masanori, c/o Canon K.K.**
**30-2, 3-chome**
**Shimomaruko, Ohta-ku, Tokyo (JP)**

(74) Representative : **Bühling, Gerhard, Dipl.-Chem.**
**Patentanwaltsbüro Tiedtke-Bühling-Kinne &**
**Partner, Bavariaring 4**
**D-80336 München (DE)**

EP 0 408 918 B1

## Description

BACKGROUND OF THE INVENTION

Field of the invention

This invention relates to a method for detecting nucleic acid utilizing the hybrid formation reaction with two kinds of nucleic acids having complementarity, and a method for detecting a gene inherent in gene disease utilizing said method.

Related Background Art

When single-stranded DNAs or RNAs have complementarity to each other, the portions having complementarity are bonded to become a double-strand, thereby forming a hybrid. As the methods for detection or quantitation of nucleic acids utilizing the hybridization reaction, there may be included various methods such as the Southern method, etc., which have become one basic technique in various genetic engineering methods such as cloning of gene, gene recombination, screening of desired gene or diagnosis of disease by use of test sample gene.

As the analytical method utilizing hybridization of nucleic acid, there has been generally employed the method, in which a probe comprising DNA or RNA is applied with a label for detection of a hybrid, the labelled probe is hybridized with a sample nucleic acid, and the hybrid formed is detected by utilizing the label imparted to the probe.

As the method for labelling probe, there have been employed the method in which a radioisotope is introduced into probe, or the method and so forth in which a compound and so forth necessary for emission reaction, color formation reaction, fluorescent reaction, etc. is introduced into or bound to probe.

As the probe, for example, nucleic acid fragments directly separated from animals, plants, microorganisms, etc., cloned nucleic acid fragments cloned according to predetermined standards, oligonucleotides synthesized by a synthesizer, etc. have been utilized depending on the purpose, etc.

With development of genetic engineering, utilization frequency and application range of the analytical method of nucleic acids utilizing hybridization are also enlarging, and it has been increasingly demanded to have a method which can perform analysis with good sensitivity by simpler operations.

For example, labelling by use of a radioisotope, while it has the advantage of having good detection sensitivity of hybrid, etc., due to handling of a radioactive substance, expensive reagents, special instruments, devices, etc. therefor are required, and also the operations are accompanied with danger.

In contrast, non-radioactive labelling as represented by labelling with an enzyme utilizing the fluorescent reaction of biotin-avidin-anti-avidin antibody-fluorescent dye complex, while it has the advantage of performing labelling and detection by safe and simple operations, has the drawback that detection sensitivity is liable to be lower when employed for labelling of probe.

Also, in various analyses, nucleic acid fragments having nucleotide chain lengths of 100 bases or more may be frequently employed as the probe to be labelled. Although such relatively longer nucleic acid fragment has the advantage that it can be labelled with relative ease, synthesis by a synthesizer can be done with difficulty, and therefore there is the drawback that cumbersome workings such as separation operation from living body, cloning, etc. are required for its preparation.

In contrast, a nucleic acid fragment with a length of less than 100 bases has the advantage that it can be synthesized easily by a synthesizing instrument. However, it has the drawback that labelling, particularly labelling by nick translation or an enzyme cannot be easily done.

Further, when it is desired to analyze plural kinds of nucleic acids by use of plural kinds of probes at the same time, for discrimination of which probe is contained in the hybrid formed, it is necessary to apply labels different from each other to the probes corresponding to the respective nucleic acids. However, such operations are extremely cumbersome.

On the other hand, application of the analytical method of nucleic acid utilizing hybridization for the detection method of a gene related to disease is now attracting attention.

As the diseases induced by mutation, etc. at gene level, there have been known various gene diseases, for example, phenylketonuria, hereditary hemoglobin abnormal diseases such as thalassemia, etc., OTC (ornitine transcarbamylase) deficiency, Duchenne type muscular dystrophy, etc.

Also, there is a report that mutation of a protein by gene mutation participates in generation of a certain kind of cancer.

Or, the probability is increasing that serious diseases such as AIDS, ATL etc. are caused to occur by retrovirus.

Diagnosis of a gene disease has been generally practiced by measuring the activity of the enzyme and the amount of the protein, both of which enzyme and protein are characteristic of the gene disease and judging where there is deficiency or abnormality in these from the results, but in some cases, the enzyme which becomes the index of the disease may exist locally at the site of organs, etc. from which sampling can be done with difficulty, and therefore a test sample may be available with difficulty, and also the index enzyme to be measured cannot be sometimes identified, and these diagnosis methods are not necessarily satisfactory.

On the other hand, diagnosis of viral infectious disease has been done exclusively by the ELISA method and the PA method, but these methods are not necessarily correct.

If a gene disease, cancer, viral infectious disease can be found at early stage by diagnosis at gene level for which sampling of a test sample is relatively easier and correct examination can be expected, adequate therapy can be done at early stage. Thus, it has been increasingly demanded to have a diagnosis method at gene level.

As one of such powerful methods, there is, for example, RFLP (restriction fragment length polymorphism).

This method is a method in which the DNA fragments obtained by cleavage of the whole gene of human being with restriction enzymes are electrophoresed on agarose gels, the electrophoresed fragment is hybridized with a probe DNA labelled with radioisotope, etc., and the electrophoresis of the hybrid detected by utilizing the label is compared with the electrophoresis pattern obtained similarly for the normal gene, thereby detecting the gene related to the disease.

The diagnosis method of gene disease by analysis of gene utilizing RFLP, etc. is attracting attention as an extremely useful method in practicing surer diagnosis.

However, in practice of these methods, the testing personnel is required to have molecular biological knowledges and skilled technique, and yet the operations are also cumbersome, and therefore those engaged in medicine such as physicians, examination engineers, etc. cannot easily practice the method.

Also, these methods require a time of about one day for each step of cleavage of DNA, Southern blotting and hybridization, and a long time is required before obtaining the final result, whereby the number of test samples handled per one examination is limited and therefore it is a very difficult working to examine DNAs of many patients over multiple items.

Also, application of the detection method utilizing hybridization of nucleic acids to the taxonomic method of microorganisms such as bacteria, etc., particularly for assay of pathogens, is attracting attention.

Taxonomic identification of microorganisms has been practiced by investigating the ecological properties and biochemical properties of the microorganisms by comparison with standard microorganisms.

However, according to such method, in some cases, depending on the examination method, judgement of the properties may differ, or the identification results may differ depending on which property is posed importance.

In the case when the test sample is bacteria and so forth obtained from a patient having caused an infectious disease, if there is an error in the identification results, there can be done no adequate corresponding treatment, and hence surer identification is particularly required.

Accordingly, in recent years, in order to provide surer identification method, there have been made attempts to use the DNA-DNA hybridization method for detection, identification of causative bacteria in bacterial infectious diseases.

This method is a method in which a characteristic base sequence of said bacteria among DNA's in bacteria is made the standard sequence, and whether a base sequence with high homology with said standard sequence exists in the DNA extracted from the test sample bacteria is examined according to the hybridization method by use of a probe capable of detecting said standard sequence, thereby performing taxonomic identification of the test sample bacteria

As the probe in this method, the DNA fragment cloned from the chromosome of bacteria, the plasmid itself possessed by the bacteria, synthetic oligonucleotides, etc. may be employed.

Specifically, there may be included the following methods, in which the probe employed is:

1) a cloned random fragment;
2) a ribosome RNA; and
3) a synthetic oligonucleotide having a base sequence characteristic of the species or the genus selected from the base sequence of ribosome RNA.

As the above method 1), a method in which random fragments obtained by cleavage of DNA extracted from bacteria with an appropriate restriction enzyme, and the clone specifically reactive only with the desired taxonomic group from among them is selected and used as the probe has been known from, for example, Criale, M.S., Flowers, R.S. et al: DNA probes, 143-148, 1986, etc.

As the above method 2), there has been known a method disclosed by Edelstein, P.H.; J. Clin. Microbiol.,

23:481-484, 1986. This method is a method which has called attention to the fact that the ribosome RNAs of bacteria are relatively similar within the same taxonomic group, and there is the advantage that the detection sensitivity becomes higher by use of ribosome RNA, because a large number of gene copies exist in the chromosome of bacteria.

As the above method 3), there have been reported examples in which base sequences specific to the genus, the species or the subspecies, etc. belonging to the genus Proteus selected from the base sequence of the ribosome RNA of the genus Proteus are utilized for probe (Haun, G. & Gobel, U.; FEMS Microbiol. Lett., 43: 187-194, 1987).

However, also these methods have commonly the problems in the various analytical methods utilizing hybridization as previously described.

EP-A-0 135 159, EP-A-0 146 815 and EP-A-0 144 914 all describe hybridisation assays wherein the hybrid, formed between the nucleic acid to be detected and the probe, is labelled after reaction has occurred. Label is conjugated to either an antibody to the hybrid (EP-A-0 135 159), two different reagents, having two different labels, which both bind specifically to the hybrid (EP-A-0 144 914), or to an antibody to an intercalating molecule (EP-A-0 146 815).

EP-A-0 235 726 discloses a method for the determination of particular nucleic acid sequences in a sample nucleic acid in which the sample itself is labelled.

## SUMMARY OF THE INVENTION

The present invention has been accomplished in view of the problems in various detection methods of the prior art utilizing the hybridization methods as described above, and its object is to provide a method for detecting nucleic acid utilizing the hybridization method which can be practiced simply and correctly.

The present invention provides a method for detecting nucleic acid including the process of reacting a sample nucleic acid with a probe nucleic acid and analyzing the presence or absence of a nucleic acid to be detected-probe nucleic acid hybrid in the reaction mixture obtained, comprising the processes of:

a) reacting a sample nucleic acid with a probe nucleic acid;

b) applying a label only to the hybrid formed in the reaction mixture obtained in the process a) by primer extension; and

c) detecting the hybrid labelled in the process b) by utilizing said label.

## BRIEF DESCRIPTION OF THE DRAWINGS

Fig. 1 is a chart showing the arrangement of spots of the probe nucleic acid in Example 2;

Fig. 2 is a chart showing the arrangement of spots of the probe nucleic acid in Example 3;

Fig. 3 is a diagram showing schematically the constitution of the nucleic acid to be detected-probe nucleic acid hybrid; and

Fig. 4A and 4B are diagrams for illustration of Example 7 and Comparative example 1.

## DESCRIPTION OF THE PREFERRED EMBODIMENTS

The present invention can be utilized for detection and quantitation of various nucleic acids such as DNA or RNA derived from living bodies of human, animals, plants, microorganisms such as bacteria, etc., fungi, protozoa, etc.,cloned DNA, recombinant DNA, synthetic DNA, etc., and the kind of the nucleic acid which is the subject to be detected (nucleic acid to be detected) is not limited.

As the probe nucleic acid, any nucleic acid having a base sequence which can hybridize effectively with the nucleic acid to be detected can be utilized, and an oligonucleotide with relatively shorter nucleotide chain length which can be artificially synthesized conveniently by a synthesizer can be readily utilized.

The nucleotide chain length of the probe nucleic acid should be preferably made 1/10 or less of the nucleic acid to be detected.

In the present invention, since a hybrid comprising a probe nucleic acid and a nucleic acid to be detected is applied with a label, the requirement necessary for labelling is not demanded for the probe nucleic acid itself.

For example, in labelling according to the nick translation method or a binding method of a labelled enzyme, the nucleic acid is required to have a nucleotide chain length of some extent or more so as to be labelled, but the probe nucleic acid to be utilized in the present invention is not demanded to have such requirement.

Therefore, and also, as described above, it becomes possible to utilize one with short nucleotide chain length capable of being readily available (prepared) and realizing detection with high sensitivity as the probe nucleic acid.

However, since the hybrid formed is applied with a label in the present invention, it is desirable to choose the constitution and the nucleotide length of the probe nucleic acid so that the hybrid can be easily labelled.

For example, as described below, in the labelling method according to the method in which one of the nucleotide chains of the double-stranded portion of the hybrid is utilized as the primer, and the nucleotide chain is extended from its end to have a labelling substance incorporated in said extended portion, when a probe nucleic acid is utilized as the primer, the hybrid to be formed is required to have a constitution so that the nucleotide chain of the nucleic acid to be detected hybridized with the probe nucleic acid can function as the template during extension of the primer end, namely that the nucleotide chain of the nucleic acid to be detected may exist under the state of a single strand in the terminal end extension direction of the probe nucleic acid.

Therefore, in the case of such labelling method utilizing a probe nucleic acid as the primer, the constitution and the nucleotide length of the probe nucleic acid should be desirably determined in view of the structure of the hybrid to be formed.

In some cases, the sample nucleic acid may be also utilized as the primer.

Hybridization of a sample nucleic acid and a probe nucleic acid can be effected following conventional methods.

Since the conditions of hybrid formation reaction depend on the nucleotide chain length and the base sequence possessed by the probe nucleic acid, the operational conditions in hybridization may be conveniently selected optimally corresponding to the desired purpose.

The hybrid formation reaction can be generally carried out in a hybridization solution containing formamide, an appropriate salt and Denhardt solution, while controlling the temperature.

For the hybrid formation reaction, there can be utilized the method in which a sample nucleic acid and a probe nucleic acid are reacted in a hybridization solution, the method in which a sample nucleic acid is reacted with a probe nucleic acid immobilized on a carrier in a hybridization solution the method in which a probe nucleic acid is reacted with a sample nucleic acid immobilized on a carrier, etc.

For immobilization of sample nucleic acid or probe nucleic acid onto a carrier, it is possible to utilize the method in which nucleic acid is bound physically or chemically onto a carrier such as nitrocellulose, nylon film, various gels, etc.

In the method of the present invention, the hybrid formed as the result of the reaction between a sample nucleic acid and a probe nucleic acid is selectively applied with a label.

As the labelling method, it is possible to utilize, for example, the method in which, when one of the chains constituting the double-stranded portion of the hybrid is utilized as the primer and its end is extended to form the single-stranded portion into a double-stranded portion, a labelling substance is incorporated in the extended portion newly synthesized.

According to this method, in the nucleic acid forming no hybrid, since there exists no portion functioning as the primer for formation of a new double-stranded and no portion which becomes the template for formation of the extended portion, there occurs no double-strand formation reaction which incorporates the above-mentioned labelling substance.

Therefore, even when the labelling reaction may be performed under the mixed state of the nucleic acids forming no hybrid and the hybrids, only the hybrids can be applied selectively with the label.

For such labelling, various methods for synthesizing new double-stranded portion can be utilized.

For example, it is possible to utilize the method in which the hybrid to be labelled is reacted with nucleotides such as dATP, dCTP, dGTP, dTTP, etc. necessary for extension of the primer end and a labelled nucleotide is employed for one or two or more of the nucleotides used during the reaction to have the label incorporated in the nucleotide chain newly formed.

As the labelled nucleotide, it is possible to utilize those utilized generally for labelling of probes, for example, those labelled with radioisotopes (RI), those label led with non-radioactive labelling substance (nonRI) such as enzymes or compounds necessary for inducing fluorescence, emission or color formation, for example, biotin, dinitrophenylnucleotide derivatives, etc.

As the enzyme for formation of nucleotide chain formation, various DNA polymerases such as E. coli DNA polymerase I, Klenow fragment of DNA polymerase I, T4 DNA polymerase, etc., and reverse transcriptases, etc. can be utilized.

Labelling in the present invention can be performed under the state where the hybrid is immobilized on an appropriate carrier, or under the state where the hybrid is dissolved or dispersed in an appropriate solvent.

After completion of the reaction for labelling, separation of the hybrid labelled and the labelling substance not incorporated into the hybrid can be performed according to such methods as described below.

(1) When an immobilized probe nucleic acid is used:

A sample nucleic acid is reacted with an immobilized probe nucleic acid immobilized on an appropriate carrier. When a hybrid is formed in this reaction, the hybrid also becomes immobilized on the carrier. When labelling treatment is further performed under such state, the carrier is washed by varying, for example, the salt concentration and the temperature, to wash away the labelling substance not incorporated in the hybrid.

(2) When an immobilized sample nucleic acid is used:

A probe nucleic acid is reacted with a sample nucleic acid immobilized on an appropriate carrier. When a hybrid is formed in this reaction, the hybrid also becomes immobilized on the carrier. Following subsequently the procedure as in the above method (1), the carrier is washed similarly to wash away the labelling substance not incorporated in the hybrid.

(3) When the reaction between a sample nucleic acid and a probe nucleic acid is carried out without immobilization:

The reaction between a probe nucleic acid and a sample nucleic acid is carried out in an appropriate medium. After the reaction mixture is subjected to labelling treatment, the hybrid and the labelling substance not incorporated in the hybrid are separated by an appropriate separation method.

As such separation method, separation by ethanol precipitaion, gel filtration column, etc. can be utilized.

When a probe nucleic acid is utilized as the primer for the reaction as described above, it is desirable to use one having a constitution or a nucleotide chain length utilizable as the primer after hybrid formation as the probe nucleic acid. In some cases, the sample nucleic acid may be also used as the primer.

Ordinarily, when the same extent of labelling amount is used, nonRI label is generally lower in sensitivity as compared with RI label, but in the method of the present invention, high sensitivity detection is rendered possible even when a nonRI label may be employed.

In the method of the prior art, probe nucleic acids with short nucleotide chain lengths are labelled and used in most cases, but in such case, the amount of the label incorporating portion is limited, and the amount of the label uncorporated is also limited.

In contrast, in the method of the present invention, by using a combination of a probe nucleic acid and a sample nucleic acid with due consideration so that the double- stranded portion newly synthesized by extension of the end of the nucleic acid which becomes the primer in the operation of labelling as described above has a enough long length, the amount of the label incorporated in labelling can be increased, whereby the low sensitivity can be also compensated with such large amount of the label incorporated even when a nonRI label may be employed to enable detection of high sensitivity.

On completion of labelling of the hybrid, the hybrid is detected by utilizing said label.

Also, by analyzing quantitatively the label amount possessed by the hybrid formed, the nucleic acid to be detected can be quantitated.

By use of plural kinds of nucleic acids as the probe nucleic acid in the operations as described above, when plural nucleic acids to be detected are contained in the sample nucleic acid, they can be detected at the same time.

Representative examples of the methods by use of plural kinds of probes are shown below.

a) The method of using immobilized probes:

First, by immobilizing plural kinds of probe nucleic acids corresponding to the respective plural kinds of nucleic acids to be detected at a predetermined arrangement so that it may be discernible which probe nucleic acid is positioned at which position, the immobilized probe nucleic acids are reacted with a sample nucleic acid.

After completion of the reaction, the treatment for applying a label on the hybrid formed is effected, and the formation position of the hybrid is detected by utilizing the label. The kind of the corresponding probe nucleic acid is identified from the position indicating positiveness, and from the result the kind of the nucleic acid to be detected contained in the sample nucleic acid can be judged.

In this method, since probe nucleic acids are immobilized at the positions previously set, it can be easily judged from the immobilized positions previously set of probe nucleic acids that with which probe nucleic acid the sample nucleic acid has been hybridized.

Therefore, it is not necessary to use different labels for respective probe nucleic acids so that the respective plural kinds of probe nucleic acids can be chosen as in the method of the prior art in which labelled probes are allowed to react with a sample nucleic acid immobilized.

For effecting the hybrid formation reaction with plural kinds of probe nucleic acids efficiently at the same time by the same operation, it is preferable that the nucleotide chain lengths of the respective probe nucleic acids should be controlled to the lengths necessary for the individual hybrid formation reactions in the plural kinds of probe nucleic acids to proceed under the same conditions.

Specifically, for example, the dissociation temperatures (Tm: temperature for single strand formation) of the hybrids formed in the individual hybrid formation reactions may be preferably made regular.

More specifically, for calculation of Tm value, various formulas have been employed depending on the hybridization solution employed. For example, in 0.9 M NaCl, Tm of an oligonucleotide of 20-mer or less is rep-

resented by:

$$Tm = 2 \times (A + T) + 4 \times (G + C)$$

(A, T, G and C respectively show the number of adenine, thymine, guanine and cytosine) (see Ryo Kinami, Mitsuru Nagahara "Continued Biochemical Experimental Course 1, Gene Study Method II" Tokyo Kagaku Doujin, p. 236).

Accordingly, following this formula, the base sequences of the respective probe nucleic acids may be substituted in this formula for selection of their lengths so that the Tm values in the respective probe nucleic acids may be regular.

The nucleotide chain lengths of the plural kinds of probe nucleic acids should be preferably made 1/10 or less of the average nucleotide chain length of the nucleic acids to be detected.

b) The method of using an independent reaction regions such as microplate, etc.:

First, solutions for hybridization containing sample nucleic acids in the respective reaction regions of a carrier having independent reaction regions such as microplate, etc. are prepared.

Next, plural kinds of probe nucleic acids are added each alone in the reaction regions to effect hybridization. In that case, it should be ascertained which probe nucleic acid is added in which reaction region.

After completion of the reaction, reactions necessary for labelling of the hybrids formed in the respective reaction regions is conducted, and then the hybrid formation is detected by utilizing said label, the kind of the corresponding probe nucleic acid is identified from the reaction region exhibiting positiveness, and from the result the kind of the nucleic acid to be detected contained in the sample nucleic acid is judged.

For effecting the hybrid formation reaction with plural kinds of probe nucleic acids efficiently at the same time by the same operation, it is preferable that the nucleotide chain lengths of the respective probe nucleic acids should be controlled to the lengths necessary for the individual hybrid formation reactions in the plural kinds of probe nucleic acids to proceed under the same conditions.

For example, as described above, the respective nucleotide chain lengths should be preferably selected so that Tm values may be regular.

The nucleotide chain lengths of the plural kinds of probe nucleic acids should be preferably made 1/10 or less of the plural nucleotide chain length of the nucleic acids to be detected.

In the method of the present invention utilizing plural kinds of probe nucleic acids including the methods, a, b as described above, it is not necessary to effect labelling of the respective probe nucleic acids with different labels for choice of the plural kinds of probe nucleic acids as in the method of the prior art wherein labelled probes are reacted with an immobilized sample nucleic acid.

On the other hand, it is also possible to perform simultaneous examination of plural kinds of sample nucleic acids by use of one kind of probe nucleic acids for plural kinds of sample nucleic acids.

For example, by immobilizing plural kinds of sample nucleic acids on an appropriate carrier so that the position of each sample nucleic acid can be discriminated, and then carrying out the reaction with one kind of probe nucleic acid and applying labelling of the hybrids formed and the treatment for detection of the label, existence of the nucleic acid to be detected can be confirmed in the sample corresponding to the position exhibiting positiveness.

Further, this method can be also performed without immobilization of nucleic acids on a carrier capable of forming independent reaction regions such as microplate, etc..

The method for detecting nucleic acid of the present invention as described above is useful for detection of genes inherent in gene diseases, cancers, viral infectious diseases.

As an example of detection of genes inherent in gene diseases, etc. according to the method of the present invention, the method including the processes shown below can be mentioned:

a) the process of immobilizing on a suitable carrier a probe nucleic acid related to the gene which is an index of the disease which is the examination item;

b) the process of reacting the immobilized probe obtained in the process a) with the test sample chromosome DNA (sample nucleic acid);

c) the process of applying the treatment necessary for labelling of the hybrid formed on the carrier after the process b);

d) the process of detecting the hybrid formed in the process b) by utilizing the label in the process c).

As the nucleic acid probe used in the above process a), one having a base sequence necessary for recognizing the base sequence inherent in a gene of gene disease, etc. may be employed, and its nucleotide length is not particularly limited.

For example, when a disease based on point mutation by substitution of one base pair is to be detected, in judging the difference of one base effectively and with good sensitivity, as the nucleic acid probe, an oligonucleotide comprising 100 bases or less, preferably 29 bases or less, more preferably 17 to 20 bases may be employed.

Also, the substitution site corresponding to point mutation should be preferably arranged so as to be positioned at the center of the oligonucleotide as the probe nucleic acid for performing more accurate judgement.

In the process a), by immobilizing the plural kinds of probe nucleic acids corresponding to the plural examination items at a predetermined arrangement on the same carrier, examination over plural items can be done at the same time. Also, in this method, the operations of the method a using plural kinds of probe nucleic acids as previously described can be applied.

As the test sample DNA in the process b), a chromosome DNA itself, a chromosome DNA cleaved to a suitable length with an appropriate restriction enzyme, etc. can be utilized.

When multiple items of examination are to be performed at the same time, it is efficient to utilize fragments cleaved to suitable lengths with a restriction enzyme.

Also, the hybrid formation reaction can be performed according to the method as previously described.

When point mutation is to be detected by this method, only one difference of nucleotide may sometimes fail to differentiate presence and absence of point mutation due to generation of mismatch between the probe and the nucleic acid.

However, since Tm of hybrid is influenced by the base pair number not complementary in the mismatched hybrid, the contents of G (guanine) and C (cytosine) in the probe nucleic acid, the nucleotide chain length of the probe nucleic acid, the position of point mutation, the kind of mismatch, etc., it is important to select the temperature conditions, etc. which are free from generation of mismatch hybrid interfering with detection of the desired point mutation, corresponding to the constitution of the probe nucleic acid employed, etc.

For example, Tm drops by 5 to 10° per one base pair which is not complementary contained in a mismatch hybrid.

Therefore, the temperature of hybridization required to be selected in view of the Tm of the desired hybrid and the Tm of the mismatch hybrid which can be formed.

Also, when plural kinds of probe nucleic acids are used as immobilized on the same carrier, in addition to the requirement concerning the mismatch as described above, the nucleotide chain lengths, etc. of these should be preferably controlled so that the Tm of the hybrids formed with the respective probe nucleic acids may be regular.

For labelling in the process c), the methods as previously mentioned, etc. can be utilized.

The process d) is performed according to the method corresponding to the label employed.

In the method as described above, prove nucleic acids are used in the form immobilized, but test sample nucleic acids may be also immobilized in place of probe nucleic acids.

More specifically, by immobilizing plural kinds of test samples so that the immobilized positions are discernible, and reacting one kind of probe nucleic acid therewith, plural kinds of test samples can be examined at the same time for one examination item.

Further, the test sample nucleic acids and probe nucleic acids may be also reacted without immobilization of both of them.

As an example of such method, the method including the following processes may be included:

1) the process of hybridizing test sample nucleic acids with a probe nucleic acid in an appropriate medium;

2) the process for applying necessary treatment for labelling of the hybrid formed on the reaction mixture obtained in the process 1);

3) the process of detecting the hybrid formed in the process 1) by utilizing the label applied in the process 2).

The operations of hybridization, labelling of the hybrid, detection of the hybrid with the label in these methods can be performed according to the operations as previously described.

Also, by performing at the same time for plural kinds of probe nucleic acids the processes 1) to 3) by using individually the plural kinds of probe nucleic acids according to the operations as described previously in the item b of the method of using plural kinds of probe nucleic acids, plural examination items can be examined at the same time.

On the other hand, after throwing plural test sample nucleic acids respectively in the reaction regions of the carrier capable of forming independent reaction region such as microplate, etc. so that the test sample placed in each reaction region may be discernible, the same kind of probe nucleic acid is added into each well, followed by hybridization, labelling of the hybrid and treatment for detection of the label, whereby it can be judged whether the sample nucleic acid corresponding to the well exhibiting positiveness is positive to the examination item or not.

In the detection method of a gene inherent in gene diseases, etc. as described above, by using as a probe a nucleic acid having a base sequence inherent in an microorganism such as bacteria, virus, etc., and a nucleic acid sepsrated from a microorganism as the sample, microorganisms can be identified taxonomically.

As the probe nucleic acid in that case, in the case of taxonomy of bacteria, one having a nucleotide chain length of about 20-mer, while in the case of taxonomy of virus, one having a nucleotide chain length of about 20-mer may be suitably employed.

Example 1

A 41-mer oligonucleotide having the following DNA base sequence constituting a part of a plasmid pUC 19 was synthesized by a DNA synthesizer (ABI, Model 381 A).

5'                                                                                                3'

GATCGCCCTTCCCAACAGTTGCGCAGCCTGAATGGCGAATG

By use of a part of the synthesized product, its purity was examined by 15 % polyacrylamide gel electrophoresis containing 7 M urea. As the result, the purity of the synthesized product was judged to be 90 % or higher, and therefore the synthesized product was used directly without purification in the subsequent operations.

A solution (50 μl) containing 50 μg of the synthesized product was mixed with 100 μl of a mixed reagent solution [0.12 M Tris containing 0.46 M potassium cacodylate (pH 6.9), 3.3 mM $CoCl_2$, and 0.33 mM dithiothreitol], 40 μl of 4.0 mM biotinylated UTP (manufactured by BRL Co.), 1 μl of 1.0 mM dTTP, 100 μl of water and 15 μl (about 90 units) of TdT (Terminal Deoxynucleotidyl Transferase), and the reaction was carried out at 30 °C for 10 minutes. The reaction was stopped by addition of 4 μl of 0.2 M EDTA.

After stopping the reaction, phenol treatment, ethanol precipitation were effected, and the precipitates obtained were dried and then dissolved in 100 μl of water.

Agarose (gel) was activated with cyanogen bromide, and avidin was bound thereto to form an immobilized phase (carrier).

The immobilized phase was added into the aqueous solution of the precipitates obtained by the treatment of the 41-mer oligonucleotide previously obtained, and these were mixed while stirring slowly for 20 minutes, followed by centrifugation treatment under gentle conditions, and the supernatant was removed to give an immobilized probe nucleic acid.

On the other hand, as sample nucleic acids, a plasmid pBR 322 (Sample A), a plasmid pUC 19 (Sample B) and a mixture of the plasmid PBR 322 and the plasmid pUC 19 (Sample C, mixing weight ratio 1:1) were prepared, these samples were digested in conventional manner with EcoRI (TOYOBO), then the digested products were subjected to heating treatment to form the double- stranded DNA into single strand, and the following operations were conducted by using reaction products individually containing the three kinds of single- stranded DNA obtained from the respective samples.

Into a test tube were introduced 20 μg of the reaction product containing the single-stranded DNA and the immobilized probe nucleic acid previously prepared, and to the mixture was added 10 μl of a 10 x annealing solution [1 x annealing solution: 100 mM Tris-HCl (pH 8.0) containing 60 mM $MgCl_2$ 60 mM β-mercaptoethanol and 500 mM NaCl], and further distilled water was added to make up the total volume to 100 μl.

The mixed solution obtained was heated at 65 °C over 10 minutes, and then the temperature was lowered to room temperature over about one hour.

Next, after addition of 10 μl of 10 x annealing solution, 10 μl of 1 mM dATP, 10 μl of 1 mM dCTP and 10 μl of 1 mM dGTP, $P^{32}$-TTP 100 Ci was added, and the total amount was made up to 200 μl with distilled water to prepare a solution for labelling.

Into the solution for labelling, 5 units of Klenow fragment (manufactured by Toyobo Co., Ltd.] were added, and the reaction was carried out under ice-cooling for one hour.

After completion of the reaction, the solid phase was separated from the reaction mixture, which was washed twice with TE buffer, and its radiation was counted by a scintillation counter.

As the result, in the solid phase finally obtained in the operation by use of Sample B and the operation by use of Sample C, radiation of about $10^8$ cpm (about $10^4$-fold of that when employing Sample A) was counted, whereby existence of pUC 19 was confirmed in these Samples B and C.

Example 2

A 41-mer oligonucleotide having the following DNA base sequence constituting a part of a plasmid pUC 19 (Probe A) and a 41-mer oligonucleotide having the following DNA base sequence constituting a part of a plasmid pBR 322 (Probe B) were synthesized by a DNA synthesizer (manufactured by ABI, Model 381 A).

Probe A

5'                                                 3'

GATCGCCCTTCCCAACAGTTGCGCAGCCTGAATGGCGAATG

Probe B

5'                                                 3'

CCGTTCAGCCCGACCGCTGCGCCTTATCCGGTAACTATCGT

When the purities of these synthesized products were examined according to the same operation as in Example 1, both were found to have a purity of 95 % or more. These synthesized products were used directly without purification in the subsequent operations.

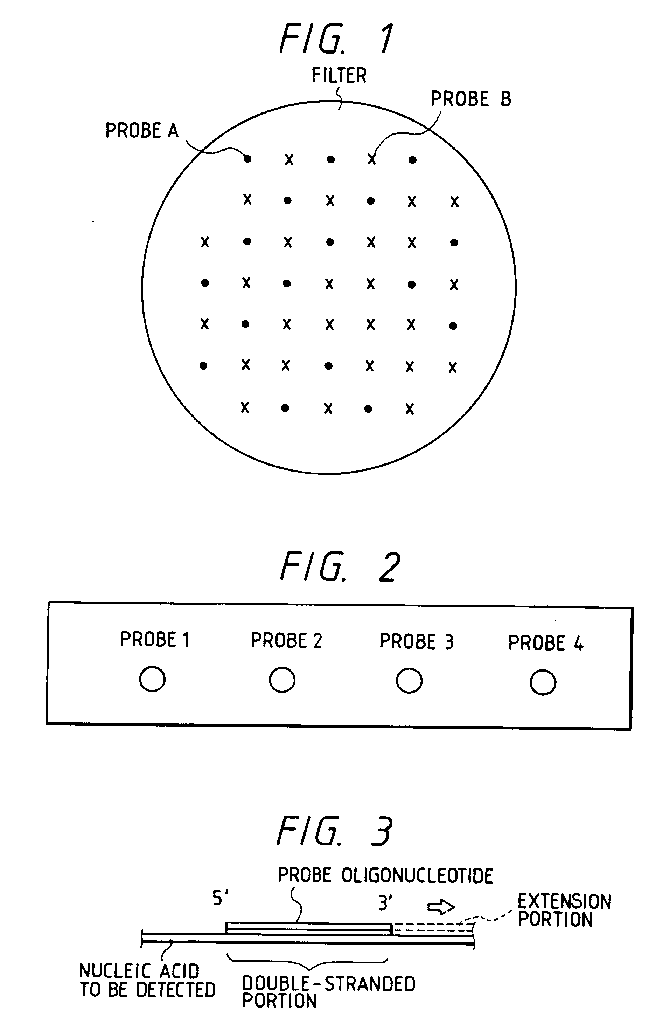

Solutions for spotting of each of the synthesized products at 200 ng/μl concentration were prepared, and each 2 μl of the solutions was spotted onto a nitrocellulose filter (Schleicher & Schanel) so that the spots of Probes A and B were arranged as shown in Fig. 1, dried and then baked at 80 °C for 2 hours.

On the other hand, as sample nucleic acids, the following samples were prepared.

Sample D:

Mixture of plasmid pUC 19 and chromosome DNA prepared from E. coli JM109 strain in conventional manner (mixing weight ratio, 1:1).

Sample E:

Mixture of plasmid pBR 322 and chromosome DNA prepared from E. coli HB101 strain in conventional manner (mixing weight ratio, 1:1).

Sample F:

Chromosome DNA prepared from E. coli HB101 strain in conventional manner.

By using these samples D to F individually, the following operations were conducted.

To 2 μl of a solution containing 1 μg of the sample, 2.0 μl of 10 x TA buffer and 16.0 μl of water were mixed, 10 units of HindIII (manufactured by Toyobo Co., Ltd.) were added to the mixture obtained and the mixture was left to stand at 37 °C for 2 hours to carry out the reaction.

After completion of the reaction, the reaction mixture was heated at 95 °C for 5 minutes to convert the double-stranded DNA into single strands and then mixed with 9 ml of 100 % formamide, 5 ml of 20 x SSC, 0.4 ml of 50 x Denhardt solution, 0.4 ml of 1 M sodium phosphate (pH 6.5) and 0.1 ml of modified salmon sperm DNA (manufactured by Sigmer) at a concentration of 50.0 mg/ml to prepare a solution for hybridization.

Next, by dipping filter having the probe nucleic acid previously prepared in each solution for hybridization, hybridization was carried out.

Specifically, the filter was dipped into 200 ml of a solution with the final concentrations of 3 x SSC/and 1 x Denhardt at 42 °C for 30 minutes to perform prehybridization, followed by sealing of these within the bag made of polyethylene so that the filter may be dipped in the solution for hybridization, and these were reacted at 42 °C for 12 hours.

After completion of the reaction, the filter taken out from the bag made of polyethylene was washed by dipping in 500 ml of 2 x SSC/0.1 % SDS at room temperature for 25 minutes, and subsequently washed similarly with 500 ml of 0.2 x SSC/0.1 % SDS at room temperature for 35 minutes.

Next, each filter after washing under the state dipped in a solution for labelling with the composition shown below was sealed within a bag made of polyethylene, and left to stand at 37 °C for 2 hours to carry out the reaction.

Solution composition for labelling:

| | |
|---|---|
| 1 mM dATP | 20 µl |
| 1 mM dGTP | 20 µl |
| 1 mM dCTP | 20 µl |
| 0.4 mM biotinylated UTP | 50 µl |
| 10 x annealing solution | 100 µl |
| Klenow fragment | 10 µl |
| Distilled water | balance |
| Total | 2 ml |

After completion of the reaction, each filter taken out was washed with TE buffer, and then the coloration reaction was carried out in conventional manner (see Bioindustry, Vol. 3, No. 6, 1989, p. 479-504, etc.).

As the result, in the filter reacted with the hybridization solution from Sample A, color formation occurred at the spotted portion of Probe A, and also color formation occurred at the spotted portion of Probe B in the filter reacted with the hybridization solution from Sample B. In contrast, in the filter reacted with the hybridization solution from Sample C, no color formation was recognized.

Example 3

Among mutations of PAH gene seen in leukocyte DNA of phenylalaniuria patient, as mutations of high frequency (* portion), the following ones have been known.

Haplotype 3

Normal:     $5'$TCCATTAACAGTAAGTAATTT$^{3'}$  (Probe 1)

Abnormal:   $5'$TCCATTAACAATAAGTAATTT$^{3'}$  (Probe 2)

                              *

Haplotype 2

Normal:     $5'$CACAATACCTCGGCCCTTCTC$^{3'}$  (Probe 3)

Abnormal:   $5'$CACAATACCTTGGCCCTTCTC$^{3'}$  (Probe 4)

Accordingly, for utilizing these four kinds of oligonucleotides as probe nucleic acids, they were synthesized by a DNA synthesizer (ABI, Model 381 A).

When the purities of the synthesized products were examined according to the same operation as in Example 1, all were found to have a purity of 95 % or more.

By using directly these synthesized products, according to the same operations as in Example 2, spots of the respective probes were formed with the arrangements shown in Fig. 2 on a nitrocellulose filter.

Next, from the cultured aminiotic fluid cells from test sample donors arbitrarily selected, a double-stranded DNA was extracted in conventional manner and digested with Pvu II, and then the double-stranded DNA was converted into single strands by the heating treatment at 100 °C for 10 minutes.

Into a vessel were added the filter having spots of each probe formed thereon and 0.5 ml of 10 x annealing solution, and further the total volume was made up to 5 ml with addition of distilled water to dip the filter into the solution.

To this was added the mixture of single-stranded DNA fragments as the test sample previously obtained,

11

and the mixture was left to stand at 65 °C for 10 minutes.

After elapse of a predetermined time, the reaction mixture was gradually left to cool over about one hour.

When a hybrid is formed by this reaction, it may be considered that a partial double strand of the probe and the test sample DNA as shown in Fig. 3 is formed.

Next, to the reaction mixture left to cool to room temperature were added 0.2 ml of 1 mM dATP, 0.2 ml of 1 mM dGTP, 0.2 ml of 1 mM dCTP, 0.5 ml of 0.4 mM biotinylated UTP, 0.5 ml of 10 x annealing solution and 3.4 ml of distilled water, and then further 16 units of Klenow fragment were added to carry out the reaction at 37 °C for one hour.

In this reaction, when a hybrid having the double-stranded portion as shown in Fig. 3 is formed, DNA is synthesized from the 3'-end of one of the chains functioning as the primer of the double-stranded portion, whereby the terminal end progresses toward the downstream of the 3'-end (in the arrowhead direction). At that time, the label is incorporated within the nucleotide chain newly formed on one side.

Next, for removal of the unreacted biotinylated UTP, the filter was washed with 2 x SSC.

When the filter after washing was subjected to color formation reaction in conventional manner, color formation occurred in the spot of Probe 2, whereby the donor of the test sample was judged to be suspicious of phenylalaninuria.

Example 4

By using individually the cultured amniotic fluid cells from several donors arbitrarily selected, the double-stranded DNA was extracted in conventional manner, digested with Pvu II and then the double-stranded DNA was converted into single strands by the heating treatment at 100 °C for 10 minutes to obtain mixtures of single-stranded DNAs from the respective donors of test samples.

Next, similarly as in Example 2, each DNA mixture (each 2 μg) was spotted onto a nitrocellulose filter so that it may be discernible which spot is from which test sample donor.

Further, into a vessel were placed a filter having the spot of each test sample DNA mixture formed thereon and 0.5 ml of 10 x annealing solution, and further the total volume was made up to 5 ml with addition of distilled water so as to dip the filter into the solution.

Into this was added 1 μg of Probe 1 obtained in Example 3, and the mixture was left to stand at 65 °C for 10 minutes.

After elapse of a predetermined time, the reaction mixture was gradually left to cool to room temperature over about one hour, and treatment for labelling, washing, treatment for coloration were conducted similarly as in Example 3.

Further, the operations as described above were performed for each of Probes 2 to 4.

As the result, the fifth spot of the filter was colored when Probe 2 was used, and the donor of the test sample of this spot was judged to be suspicious of phenylalaninuria.

Example 5

Probes 1 to 4 (each about 1 μg) synthesized in Example 3 were added dropwise onto a microplate so that it was discernible which probe was contained in which well.

Next, from the cultured amniotic cells from the test sample donors arbitrarily selected, the double-stranded DNA was extracted in conventional manner, digested with Pvu II (manufactured by Toyobo Co., Ltd.), and the double-stranded DNA was dissociated into single strands by the heating treatment at 100 °C for 10 minutes to obtain mixtures containing single-stranded DNA's of test samples.

The DNA mixtures were thrown into the respective wells in amounts each of 50 ng.

Further, after addition of 7 μl of 10 x annealing solution into each well, distilled water was added to make up an amount in each well of 70 μl.

The microplate under this state was left to stand at 65 °C for 10 minutes, and then left to cool gradually to room temperature over one hour.

Next, into each well were added and mixed 5 μl of 1 mM dATP, 5 μl of 1 mM dGTP, 5 μl of 1 mM dCTP, 8 μl of 0.4 mM biotinylated UTP, 7 μl of 10 x annealing solution and 40 μl of distilled water, and then further 10 units of Klenow fragment were added to carry out the reaction at 37 °C for one hour, thereby effecting labelling.

After completion of the reaction, 350 μl of ethanol was added into each well, and after cooling at - 70 °C for one hour, the supernatant from each well was discarded by aspiration to remove the unreacted biotinylated UTP together with the supernatant.

Next, after drying of the microplate, blocking was effected in conventional manner with bovine serum albumin (manufactured by Sigmer), and then a streptoavidin-alkaliphophatase solution (manufactured by BRL)

EP 0 408 918 B1

was added into each well to carry out the reaction at 37 °C.

After 30 minutes, the liquid was discarded by aspiration from each well, and further after washing of the well with 0.1 M Tris-HCl (pH 9.5)-0.1 M NaCl-50 mM MgCl$_2$ solution (buffer A), 200 $\mu$l of a mixed solution of 30 $\mu$l of BCIP solution (BRL), 44 $\mu$l of NBT solution (manufactured by BRL) and 10 ml of buffer A was added into each well to carry out the reaction at room temperature for 30 minutes, followed by judgement of color formation by a plate reader.

As the result, strong color formation was recognized in the well of Probe 2, and the donor of this test sample was judged to be suspicious of phenylalaninuria.

Example 6

A 41-mer oligonuleotide having the DNA base sequence shown below constituting a part of a plasmid pUC 19 was synthesized by a DNA synthesizer (manufactured by ABI, Model 381 A).

5'                                                                              3'

GATCGCCCTTCCCAACAGTTGCGCAGCCTGAATGGCGAATG

When the purity was examined by use of a part of the synthesized product by 15 % polyacrylamide gel electrophoresis containing 7 M urea, it was judged to have a purity of 90 % or higher and therefore the synthesized product was used as the probe nucleic acid in the subsequent operations without purification.

On the other hand, as sample nucleic acids, a plasmid pBR 322 (Sample A), a plasmid pUC 19 (Sample B) and a mixture of the plasmid pBR 322 and the plasmid pUC 19 (Sample C, mixing weight ratio 1:1) were prepared, and after these samples were digested in conventional manner with EcoRI (manufactured by Toyobo Co., Ltd.), the digested products were subjected to heating treatment to convert the double-stranded DNA into single strands, and the following operations were conducted by using individually the reaction products containing the three kinds of single-stranded DNA obtained from the respective samples.

Into a reaction tube were introduced 20 $\mu$g of the reaction product containing the single-stranded DNA and 20 $\mu$l of the probe nucleic acid previously prepared, and to the mixture was added 10 $\mu$l of a 10 x annealing solution [1 x annealing solution: 100 mM Tris-HCl (pH 8.0) containing 60 mM MgCl$_2$, 60 mM $\beta$-mercaptoethanol and 500 mM NaCl], and further the total volume was made up to 100 $\mu$l with addition of distilled water.

The mixed solution was heated at 65 °C over 10 minutes, and then the temperature was gradually lowered to room temperature over about one hour.

Next, to the reaction mixture obtained, were added 10 $\mu$l of 10 x annealing solution, 10 $\mu$l of 1 mM dATP, 10 $\mu$l of 1 mM dCTP and 10 $\mu$l of 1 mM dGTP, and then P$^{32}$-TTP 100 Ci was added, and the total amount was made up with distilled water to 200 $\mu$l to prepare a solution for labelling.

Into the solution for labelling were added 5 units of Klenow fragment (manufactured by Toyobo Co., Ltd.), and the reaction was carried out under ice-cooling for one hour.

After completion of the reaction, by use of GENECLEAN™ (manufactured by BIO 101], the unreacted P$^{32}$-TTP and the hybrid formed in the reaction mixture were separated as described below.

The silica matrix of GENECLEAN (5 $\mu$l) was added into 200 $\mu$l of the reaction mixture, and then the mixture was well stirred and maintained in ice for 10 minutes, followed by centrifugation for 5 seconds to remove the supernatant and give the pellets of the silica matrix/hybrid bound product.

At that time, the unreacted P$^{32}$-TTP was separated together with the supernatant from the hybrid bound to the silica matrix.

The pellets obtained in the separated liquid of GENECLEAN were washed three times, 3 $\mu$l of pure water was added thereto, incubation was carried out at 50 °C for 2 minutes, and the hybrid was eluted from the pellets to recover the supernatant containing the hybrid by centrifugation.

The supernatant recovered was subjected to scintillation counter for counting its radiation.

As the result, a radiation of about 10$^7$ cpm (about 10$^3$-fold of that when using Sample A) was counted in the solutions finally obtained in the operations by use of Sample B and the operations by use of Sample C, whereby pUC 19 was confirmed to exist in these Samples B and C.

Example 7

An oligonucleotide constituting a part of pUC 19 shown below was synthesized and used as such as the probe.

13

5'                                                                                              3'

ATCGCCCTTCCCAACAGTTGCGCAGCCTGAATGGCGAAT

The probe (hereinafter referred to as Probe A) was introduced into 10 microplate wells as the total of the group I: No. 1 - No. 10 and 10 microplate wells as the total of the group II: No. 1 - No. 10 each in an amount of 1 μg.

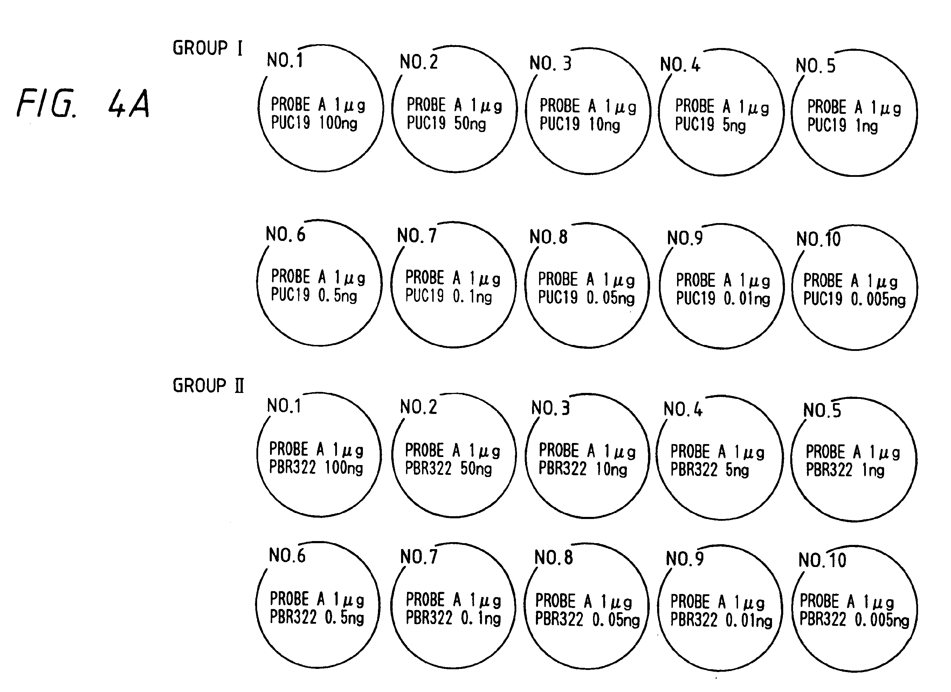

Next, the sample pUC 19 dissociated into single strands was thrown into the respective microplate wells of the group I: No. 1 - No. 10 at the ratios shown below in Table 1, and also as Control pBR 322 dissociated into single strands was thrown into the respective microplate wells of the group II: No. 1 - No. 10 at the ratios shown below in Table 1 (see Fig. 4A).

Table 1

| NO. | 1 | 2 | 3 | 4 | 5 | 6 | 7 | 8 | 9 | 10 |
|---|---|---|---|---|---|---|---|---|---|---|
| DNA amount (ng) | 100 | 50 | 10 | 5 | 1 | 0.5 | 0.1 | 0.05 | 0.01 | 0.005 |

Further, into each well was added 7 μl of 10 x annealing solution, followed by addition of distilled water to make up the amount within each well to 70 μl.

The microplate under this state was left to stand at 65 °C for 10 minutes, and left to cool gradually to room temperature over about one hour.

Next, into each well were added and mixed 5 μl of 1 mM dATP, 5 μl of 1 mM dGTP, 5 μl of 1 mM dCTP, 8 μl of 0.5 mM biotinylated UTP, 7 μl of 10 x annealing solution and 40 μl of distilled water, and then further 10 units of Klenow fragment were added to carry out the reaction at 37° C for one hour, thereby effecting labelling.

After completion of the reaction, 350 μl of ethanol was added into each well, and after cooling at - 70 °C for one hour, the supernatant from each well was discarded by aspiration to remove the unreacted biotinylated UTP together with the supernatant.

Next, after drying of the microplate, blocking was performed in conventional manner with bovine serum albumin (manufactured by Sigmer), and then a streptoavidin-alkaliphophatase solution (manufactured by BRL) was added into each well to carry out the reaction at 37 °C.

After 30 minutes, the liquid was discarded by aspiration from each well, and further after washing of the well with O.1 M Tris-HCl (pH 9.5)-0.1 M NaCl-50 mM MgCl$_2$ solution (buffer A), 200 μl of a mixed solution of 30 μl of BCIP solution (manufactured by BRL), 44 μl of NBT solution (manufactured by BRL) and 10 ml of buffer A was added into each well to carry out the reaction at room temperature for 30 minutes, followed by judgement of color formation by a plate reader.

As the result, in the group I, color formation occurred to 0.1 ng of No. 7. No color formation was seen in Control of the group II.

Comparative example 1

A double-stranded DNA constituting a part of pUC 19 shown below was synthesized, biotinylated UTP was introduced at 3'-end with T4 polymerase, followed by labelling, and the labelled product was dissociated into single strands, which were used as the probe for detection according to the prior art method.

5'                                                                                              3'

ATCGCCCTTCCCAACAGTTGCGCAGCCTGAATGGCGAAT

TAGCGGGAAGGGTTGTCAACGCGTCGGACTTACCGCTTA

14

The probe (hereinafter referred to as Probe B) was introduced into 10 microplate wells as the total of the group III: No. 1 - No. 10 and 10 microplate wells as the total of the group IV: No. 1 - No. 10 each in an amount of 1 µg.

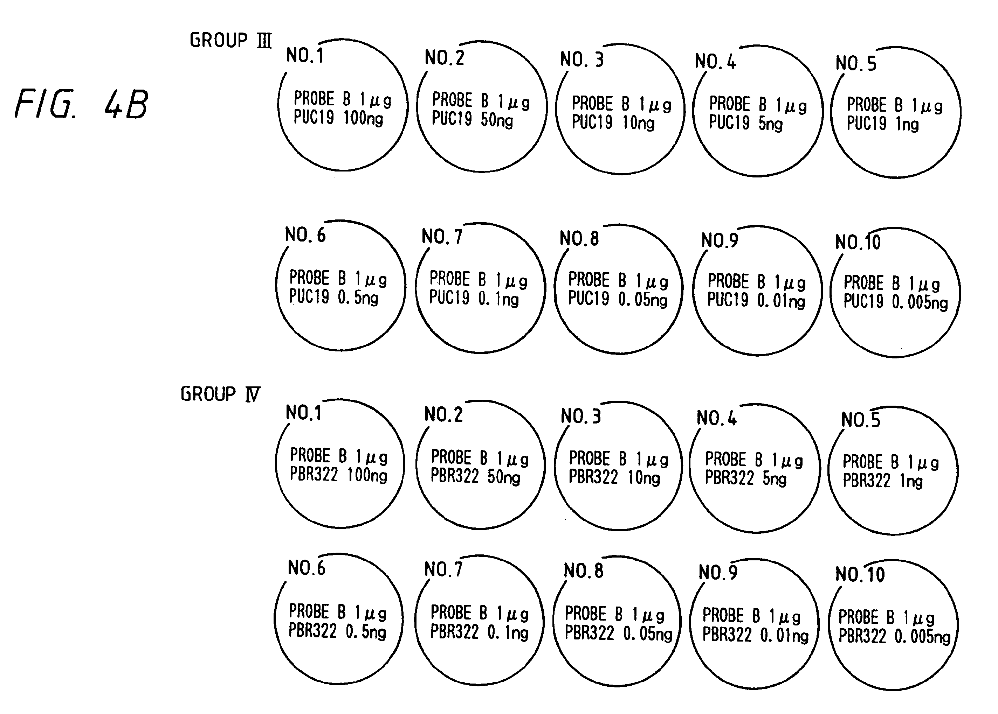

Next, the sample pUC 19 dissociated into single strands was thrown into the respective microplate wells of group III: No. 1 - No. 10 at the same ratios as in Table 1 of Example 7, and also as Control pBR 322 dissociated into single strands was thrown into the respective microplate wells of the group IV: No. 1 - No. 10 at the same ratios as in Table 1 of Example 7 (see Fig. 4B).

Further, into each well was added 7 µl of 10 x annealing solution, followed by addition of distilled water to make up the solution amount within each well to 70 µl.

The microplate under this state was left to stand at 65 °C for 10 minutes, and then left to cool gradually to room temperature over about one hour.

After completion of the reaction, 350 µl of ethanol was added into each well, and after cooling at - 70 °C for one hour, the supernatant from each well was discarded by aspiration to remove the unreacted probe together with the supernatant.

Next, after drying of the microplate, blocking was effected in conventional manner with bovine serum albumin (manufactured by Sigmer), and then a streptoavidin-alkaliphosphatase solution (manufactured by BRL] was added into each well to carry out the reaction at 37 °C.

After 30 minutes, the liquid was discarded by aspiration from each well, and further after washing of the well with O.1 M Tris-HCl (pH 9.5)-0.1 M NaCl-50 mM $MgCl_2$ solution (buffer A), 200 µl of a mixed solution of 30 µl of BCIP solution (manufactured by BRL), 44 µl of NBT solution (manufactured by BRL) and 10 ml of buffer A was added into each well to carry out the reaction at room temperature for 30 minutes, followed by judgement of color formation by a plate reader.

As the result, in the group III, color formation occurred to 5 ng. No color formation was seen in Control of the group IV.

From Example 7 and Comparative example 1, it could be confirmed that detection sensitivity was improved by about 50- fold according to the method of the present invention as compared with the prior art example. This may be considered to be due to the fact that the biotinylated UTP is incorporated in the extension reaction after annealing in an amount more by about 50-fold.

In the present invention, since no labelled probe nucleic acid is used, no requirement for labelling of the probe nucleic acid is demanded. As the result, one with a short nucleotide chain length which is readily available and also can be expected to effect detection of high sensitivity can be utilized, and detection with good sensitivity can be done.

Also, in the present invention, since the hybrid formed is applied with a label, it becomes possible to effect efficient labelling even when a non-radioactive label may be employed, whereby the labelling and detecting operations become safer and simpler.

Besides, while all of the probe nucleic acids, namely even those not participating in formation of hybrids are applied with labels in the method of the prior art which label probe nucleic acids, in the method of the present invention, only the hybrids formed are labelled, whereby the labelling substance can be saved.

Further, when detection of plural number of nucleic acids are performed at the same time by use of plural kinds of probe nucleic acids, in the method of labelling probe nucleic acids of the prior art, cumbersome operations of performing differentiation of plural number of probe nucleic acids by use of plural different kinds of labels have been required, but according to the present invention, labelling of the hybrid formed can be effected with one kind of label, whereby labelling operations can be simplified and detections of plural kinds of nucleic acids can be done extremely simply and efficiently.

A method for detecting nucleic acid including the process of reacting a sample nucleic acid with a proe nucleic acid and analyzing presence or absence of a nucleic acid to be detected-probe nucleic acid hybrid into the reaction mixture obtained comprises the processes of: a) reacting a sample nucleic acid with a probe nucleic acid; b) applying a label only onto the hybrid formed in the reaction mixture obtained in the process a); and c) detecting the hybrid labelled in the process b) by utilizing said label. An immobilized probe for nucleic acid detection to be used for the method for detecting nucleic acid according to claim 2 comprises a probe nucleic acid immobilized on a carrier, wherein said probe nucleic acid is capable of hybridizing with a nucleic acid to be detected.

## Claims

1.   A method for detecting nucleic acids including the process of reacting a sample nucleic acid with a probe nucleic acid and analyzing the presence or absence of a nucleic acid to be detected-probe nucleic acid hybrid in the reaction mixture obtained, comprising the processes of:

a) reacting a sample nucleic acid with a probe nucleic acid;

b) applying a label only to the hybrid formed in the reaction mixture obtained in the process a) by primer extension; and

c) detecting the hybrid labeled in the process b) by utilizing said label.

2. A method for detecting nucleic acids according to claim 1, wherein the probe nucleic acid or the sample nucleic acid is immobilized on to a carrier.

3. A method for detecting nucleic acids according to claim 1 or claim 2, wherein the nucleotide chain length of the probe nucleic acid is 1/10 or less of the nucleotide chain length of the nucleic acid to be detected.

4. A method for detecting nucleic acids according to claim 2, wherein plural kinds of probe nucleic acids are immobilized at predetermined arrangements on the carrier.

5. A method for detecting nucleic acids according to claim 2, wherein plural kinds of sample nucleic acids are immobilized at the predetermined arrangements on the carrier and reacted with one kind of probe nucleic acid in the process a).

6. A method for detecting nucleic acids according to claim 4, wherein the respective probe nucleic acids have nucleotide chain lengths necessary for the hybrid formation reactions in the respective probe nucleic acids to proceed under the same conditions.

7. A method for detecting nucleic acids according to any one of claims 4 to 6, wherein the nucleotide length of the probe nucleic acid is 1/10 or less of the nucleotide chain length of the nucleic acid to be detected.

8. A method for detecting nucleic acids according to any one of claims 1 to 7, wherein the nucleic acid to be detected has sequences inherent in a gene disease.

9. A method for detecting nucleic acids according to claim 8, wherein the nucleotide chain length of the probe nucleic acid is 100 bases or less.

10. A method for detecting nucleic acids according to claim 8, wherein the probe nucleic acid is an oligonucleotide with a nucleotide chain length of 25 bases or less capable of detecting the point mutation of a gene disease.

11. A method for detecting nucleic acids according to claim 1, wherein four kinds of nucleotides different from one another are used in the process b), at least one of which kinds has been labeled.

12. A method for detecting nucleic acids according to claim 11, wherein said four kinds of nucleotides consist of dATP, dCTP, dGTP and dTTP.

13. A method for detecting nucleic acids according to claim 11, wherein at least one of said nucleotides has been labeled by at least any one selected from a group consisted of radioisotope, biotin and dinitrophenyl.

14. A nucleic acid detecting kit for reacting a sample nucleic acid with a probe nucleic acid and analyzing presence or absence of a nucleic acid to be detected - probe nucleic acid hybrid in the reaction mixture obtained, comprising

e) a prove nucleic acid functioning as a primer when the hybrid is formed from the prove nucleic acid and a nucleic acid to be detected;

f) a plurality of nucleoside triphosphates, wherein at least one of said nucleoside triphosphates is modified to contain a detectable label; and

g) a primer-dependent nucleic acid polymerase.

**Patentansprüche**

1. Verfahren zum Nachweis von Nukleinsäuren einschließlich des Verfahrens der Reaktion einer Proben-Nukleinsäure mit einer Sonden-Nukleinsäure und der Analyse der Anwesenheit oder Abwesenheit eines Hybrids aus einer nachzuweisenden Nukleinsäure und einer Sonden-Nukleinsäure in der gewonnenen Reaktionsmischung, umfassend folgende Verfahrensschritte:

a) die Reaktion einer Proben-Nukleinsäure mit einer Sonden-Nukleinsäure;

b) die Anbringung einer Markierung durch Primererweiterung nur an den Hybriden, die in der im Verfahren a) gewonnenen Reaktionsmischung gebildet wurden; und

c) der Nachweis des im Verfahren b) markierten Hybrids unter Verwendung der Markierung.

2. Verfahren zum Nachweis von Nukleinsäuren nach Anspruch 1 **dadurch gekennzeichnet**, daß die Sonden-Nukleinsäure oder die Proben-Nukleinsäure auf einem Träger immobilisiert wird.

3. Verfahren zum Nachweis von Nukleinsäuren nach Anspruch 1 oder 2 **dadurch gekennzeichnet,** daß die Nukleotidkettenlänge der Sonden-Nukleinsäure 1/10 oder kleiner als die Nukleotidkettenlänge der nachzuweisenden Nukleinsäure ist.

4. Verfahren zum Nachweis von Nukleinsäuren nach Anspruch 2 **dadurch gekennzeichnet,** daß viele Sonden-Nukleinsäurearten mit vorbestimmter Anordnung auf einem Träger immobilisiert werden.

5. Verfahren zum Nachweis von Nukleinsäuren nach Anspruch 2 **dadurch gekennzeichnet,** daß viele Proben-Nukleinsäurearten mit vorbestimmter Anordnung auf einem Träger immobilisiert werden und nach Verfahrensschritt a) mit einer Sonden-Nukleinsäureart umgesetzt werden.

6. Verfahren zum Nachweis von Nukleinsäuren nach Anspruch 4 **dadurch gekennzeichnet,** daß die entsprechenden Sonden-Nukleinsäuren eine Nukleotidkettenlänge besitzen, die benötigt wird, damit die Hybridbildungsreaktionen mit den entsprechenden Sonden-Nukleinsäuren bei den gleichen Bedingungen ablaufen.

7. Verfahren zum Nachweis von Nukleinsäuren nach einem der Ansprüche 4 bis 6 **dadurch gekennzeichnet,** daß die Nukleotidkettenlänge der Sonden-Nukleinsäure 1/10 oder kleiner als die Nukleotidkettenlänge der nachzuweisenden Nukleinsäure ist.

8. Verfahren zum Nachweis von Nukleinsäuren nach einem der Ansprüche 1 bis 7 **dadurch gekennzeichnet,** daß die nachzuweisende Nukleinsäure Sequenzen besitzt, die mit einer Erbkrankheit verbunden sind.

9. Verfahren zum Nachweis von Nukleinsäuren nach Anspruch 8 **dadurch gekennzeichnet,** daß die Nukleotidkettenlänge der Sonden-Nukleinsäure 100 Basen oder weniger ist.

10. Verfahren zum Nachweis von Nukleinsäuren nach Anspruch 8 **dadurch gekennzeichnet,** daß die Sonden-Nukleinsäure ein Oligonukleotid mit einer Nukleotidkettenlänge von 25 Basen oder weniger ist, das die Punktmutation einer Erbkrankheit nachweisen kann.

11. Verfahren zum Nachweis von Nukleinsäuren nach Anspruch 1 **dadurch gekennzeichnet,** daß vier voneinander verschiedene Nukleotidarten im Verfahrensschritt b) verwendet werden, wobei mindestens eine Art markiert wurde.

12. Verfahren zum Nachweis von Nukleinsäuren nach Anspruch 11 **dadurch gekennzeichnet,** daß die vier Nukleotidarten aus dATP, dCTP, dGTP und dTTP bestehen.

13. Verfahren zum Nachweis von Nukleinsäuren nach Anspruch 11 **dadurch gekennzeichnet,** daß mindestens eines der Nukleotide mit mindes-tens einer Markierungssubstanz, ausgewählt aus Radioisotopen, Biotin und einer Dinitrophenylgruppe markiert wurde.

14. Nukleinsäure-Nachweisset für die Reaktion einer Proben-Nukleinsäure mit einer Sonden-Nukleinsäure und für die Analyse der Anwesenheit oder Abwesenheit eines Hybrids aus einer nachzuweisenden Nukleinsäure und einer Sonden-Nukleinsäure in der gewonnenen Reaktionsmischung, umfassend:

e) eine als Primer wirkende Sonden-Nukleinsäure, wenn das Hybrid aus einer Sonden-Nukleinsäure und einer nachzuweisenden Nukleinsäure gebildet wird;

f) eine Vielzahl von Nukleosidtriphosphaten, wobei mindestens eines der Nukleosidtriphosphate unter Einbau einer nachweisbaren Markierung modifiziert wurde; und

g) eine primerabhängige Nukleinsäure-Polymerase.

## Revendications

1. Procédé de détection d'acides nucléiques, comprenant l'opération de réaction d'un acide nucléique d'échantillon avec un acide nucléique servant de sonde et d'analyse de la présence ou de l'absence d'un hybride acide nucléique à détecter-acide nucléique de sonde dans le mélange réactionnel obtenu, comprenant les étapes opératoires :
   a) de réaction d'un acide nucléique d'échantillon avec un acide nucléique de sonde ;
   b) d'application d'un marqueur seulement sur l'hybride formé dans le mélange réactionnel obtenu dans l'étape opératoire a) par allongement d'amorce ; et
   c) de détection de l'hybride marqué dans l'étape opératoire b) par utilisation dudit marqueur.

2. Procédé de détection d'acides nucléiques suivant la revendication 1, dans lequel l'acide nucléique de sonde ou l'acide nucléique d'échantillon est immobilisé sur un support.

3. Procédé de détection d'acides nucléiques suivant la revendication 1 ou la revendication 2, dans lequel la longueur de chaîne de nucléotides de l'acide nucléique de sonde est égale ou inférieure à 1/10 de la longueur de chaîne de nucléotides de l'acide nucléique à détecter.

4. Procédé de détection d'acides nucléiques suivant la revendication 2, dans lequel plusieurs types d'acides nucléiques de sonde sont immobilisés à des emplacements prédéterminés sur le support.

5. Procédé de détection d'acides nucléiques suivant la revendication 2, dans lequel plusieurs types d'acides nucléiques d'échantillon sont immobilisés aux emplacements prédéterminés sur le support et amenés à réagir avec un type d'acide nucléique de sonde dans l'étape opératoire a).

6. Procédé de détection d'acides nucléiques suivant la revendication 4, dans lequel les acides nucléiques de sonde respectifs possèdent des longueurs de chaîne de nucléotides nécessaires pour que les réactions de formation d'hybrides dans les acides nucléiques de sonde respectifs s'effectuent dans les mêmes conditions.

7. Procédé de détection d'acides nucléiques suivant l'une quelconque des revendications 4 à 6, dans lequel la longueur de nucléotides de l'acide nucléique de sonde est égale ou inférieure à 1/10 de la longueur de la chaîne de nucléotides de l'acide nucléique à détecter.

8. Procédé de détection d'acides nucléiques suivant l'une quelconque des revendications 1 à 7, dans lequel l'acide nucléique à détecter possède des séquences propres à une maladie génétique.

9. Procédé de détection d'acides nucléiques suivant la revendication 8, dans lequel la longueur de chaîne de nucléotides de l'acide nucléique de sonde est égale ou inférieure à 100 bases.

10. Procédé de détection d'acides nucléiques suivant la revendication 8, dans lequel l'acide nucléique de sonde est un oligonucléotide ayant une longueur de chaîne de nucléotides égale ou inférieure à 25 bases, capable de détecter la mutation ponctuelle d'une maladie génétique.

11. Procédé de détection d'acides nucléiques suivant la revendication 1, dans lequel quatre types de nucléotides différents les uns des autres sont utilisés dans l'étape opératoire b), au moins l'un de ces types ayant été marqué.

12. Procédé de détection d'acides nucléiques suivant la revendication 11, dans lequel les quatre types de nucléotides consistent en dATP, dCTP, dGTP et dTTP.

13. Procédé de détection d'acides nucléiques suivant la revendication 11, dans lequel au moins l'un des nucléotides a été marqué avec au moins un marqueur quelconque choisi dans le groupe consistant en un radio-isotope, la biotine et le groupe dinitrophényle.

14. Kit de détection d'acides nucléiques pour la réaction d'un acide nucléique d'échantillon avec un acide nucléique de sonde et l'analyse de la présence ou de l'absence d'un hybride acide nucléique à détecter-acide nucléique de sonde dans le mélange réactionnel obtenu, comprenant
   e) un acide nucléique de sonde jouant le rôle d'amorce lorsque l'hybride est formé à partir de l'acide nucléique de sonde et un acide nucléique à détecter ;

f) plusieurs nucléoside-triphosphates, au moins l'un de ces nucléoside-triphosphates étant modifié de manière à renfermer un marqueur détectable ; et
g) une acide nucléique-polymérase dépendant d'une amorce.

# FIG. 1

FILTER

PROBE A

PROBE B

# FIG. 2

PROBE 1  PROBE 2  PROBE 3  PROBE 4

# FIG. 3

PROBE OLIGONUCLEOTIDE

5'  3'  ⇨  EXTENSION PORTION

NUCLEIC ACID TO BE DETECTED

DOUBLE-STRANDED PORTION

EP 0 408 918 B1

FIG. 4A

GROUP I

NO.1
PROBE A 1μg
PUC19 100ng

NO.2
PROBE A 1μg
PUC19 50ng

NO.3
PROBE A 1μg
PUC19 10ng

NO.4
PROBE A 1μg
PUC19 5ng

NO.5
PROBE A 1μg
PUC19 1ng

NO.6
PROBE A 1μg
PUC19 0.5ng

NO.7
PROBE A 1μg
PUC19 0.1ng

NO.8
PROBE A 1μg
PUC19 0.05ng

NO.9
PROBE A 1μg
PUC19 0.01ng

NO.10
PROBE A 1μg
PUC19 0.005ng

GROUP II

NO.1
PROBE A 1μg
PBR322 100ng

NO.2
PROBE A 1μg
PBR322 50ng

NO.3
PROBE A 1μg
PBR322 10ng

NO.4
PROBE A 1μg
PBR322 5ng

NO.5
PROBE A 1μg
PBR322 1ng

NO.6
PROBE A 1μg
PBR322 0.5ng

NO.7
PROBE A 1μg
PBR322 0.1ng

NO.8
PROBE A 1μg
PBR322 0.05ng

NO.9
PROBE A 1μg
PBR322 0.01ng

NO.10
PROBE A 1μg
PBR322 0.005ng

# FIG. 4B

**GROUP III**

NO.1
PROBE B 1μg
PUC19 100ng

NO.2
PROBE B 1μg
PUC19 50ng

NO. 3
PROBE B 1μg
PUC19 10ng

NO. 4
PROBE B 1μg
PUC19 5ng

NO. 5
PROBE B 1μg
PUC19 1ng

NO. 6
PROBE B 1μg
PUC19 0.5ng

NO. 7
PROBE B 1μg
PUC19 0.1ng

NO. 8
PROBE B 1μg
PUC19 0.05ng

NO. 9
PROBE B 1μg
PUC19 0.01ng

NO.10
PROBE B 1μg
PUC19 0.005ng

**GROUP IV**

NO.1
PROBE B 1μg
PBR322 100ng

NO.2
PROBE B 1μg
PBR322 50ng

NO. 3
PROBE B 1μg
PBR322 10ng

NO. 4
PROBE B 1μg
PBR322 5ng

NO.5
PROBE B 1μg
PBR322 1ng

NO.6
PROBE B 1μg
PBR322 0.5ng

NO.7
PROBE B 1μg
PBR322 0.1ng

NO.8
PROBE B 1μg
PBR322 0.05ng

NO.9
PROBE B 1μg
PBR322 0.01ng

NO.10
PROBE B 1μg
PBR322 0.005ng